(19) Europäisches Patentamt
European Patent Office
Office européen des brevets

(11) Publication number : **0 446 971 B1**

(12) # EUROPEAN PATENT SPECIFICATION

(45) Date of publication of patent specification : **27.07.94 Bulletin 94/30**

(51) Int. Cl.$^5$ : **C07C 303/02,** C07C 309/03, C07C 309/10, C07C 309/13

(21) Application number : **91200036.1**

(22) Date of filing : **10.01.91**

(54) **Process for the preparation of substituted sulphonic acids and/or sulphonates.**

(30) Priority : **12.01.90 GB 9000719**

(43) Date of publication of application : **18.09.91 Bulletin 91/38**

(45) Publication of the grant of the patent : **27.07.94 Bulletin 94/30**

(84) Designated Contracting States : **BE DE ES FR GB IT NL SE**

(56) References cited :
**EP-A- 0 293 913**
**EP-A- 0 351 928**
**US-A- 4 248 793**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Stapersma, Johan**
**Badhuisweg 3**
**NL-1031 CM Amsterdam (NL)**
Inventor : **Van Ginkel, Roelof**
**Badhuisweg 3**
**NL-1031 CM Amsterdam (NL)**
Inventor : **Deuling, Hendrikus Hyacinthus**
**Badhuisweg 3**
**NL-1031 CM Amsterdam (NL)**

**EP 0 446 971 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention relates to the preparation of beta-substituted sulphonic acids and/or sulphonates and to beta-substituted sulphonic acids and/or sulphonates as novel compounds.

In European Patent Application 293913 is disclosed a process for the preparation of substituted ethane sulfon compounds, in which process a mixture of dioxane and an olefin, dissolved in an inert solvent is contacted with sulphur trioxide in a falling film reactor and in which process the resulting 1,2-sultone is further reacted with an alcohol or an amine. The substituted ethane sulfon compounds are suitable as surface-active agents. In the process according to European Patent Application 293913 the use of an $SO_3$-dioxane complex is essential in order to obtain a good yield of 1,2-sultone.

It is inevitable that traces of dioxane remain in the reaction products. For a number of applications of the surface active agent the presence of dioxane is nocuous, even at very low concentrations.

Applicant has found that the use of dioxane can be avoided.

The invention relates to a process for the preparation of beta-substituted sulphonic acids and/or sulphonates which comprises reacting a beta-sultone of an internal olefin having the chemical formula

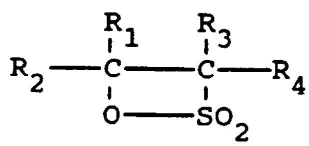 ,

wherein each of the groups $R_1$, $R_2$, $R_3$ and $R_4$ independently are linear or branched alkyl groups or hydrogen and the total number of carbon atoms of $R_1$, $R_2$, $R_3$ and $R_4$ is from 6 to 24, and at least one of $R_1$ and $R_2$ and one of $R_3$ and $R_4$ is an alkyl group, with a nucleophile, with the proviso that the nucleophile is not $H_2O$ or hydroxide ion.

A nucleophile is a molecule capable of attacking a positive centre or a positively polarized site in e.g. another molecule.

Nucleophiles are, for instance, aliphatic or aromatic (thio)alcohols or alkoxides thereof, (capped) polyethylene glycols or propylene glycols or their alkoxides, aliphatic or aromatic amines or the amides thereof, ammonia or heterocyclic nitrogen compounds.

In the process according to the invention the reaction between the nucleophile and the sultone is preferably performed in the absence of dioxane.

In Applicant's earlier European patent application 89201907 is described a process for the preparation of internal olefin sulphonates by reacting in a film reactor an internal olefin having from 8 to 26 carbon atoms with a sulphonating agent, in a mol ratio of sulphonating agent to internal olefin of 1:1 to 1.25:1, while cooling the reactor with a cooling means having a temperature not exceeding 35 °C, and allowing to neutralize and hydrolyze the reaction product of the sulphonating step. This reaction performs quite well in the absence of dioxane.

The sulphonation of the internal olefins is preferably carried out with sulphur trioxide. A falling film reactor is preferred. The cooling means is preferably water having a temperature not exceeding 35 °C, more preferably in the range of from 0 °C to 25 °C. Lower temperatures may be used, depending upon the circumstances. It is further preferred that the reaction mixture comprising the beta-sultone, is not aged during a substantial time. The reaction mixture can better not be aged at all, since then the highest percentage of beta-sultones is obtained.

This sulphonation may be carried out batchwise, semi-continuously or continuously. The reaction is preferably performed in a falling film reactor which is cooled by flowing a cooling means at the outside walls of the reactor. At the inner walls of the reactor the internal olefin flows in downward direction. $SO_3$ is diluted with nitrogen, air or any other gas, inert under the reaction conditions. The concentration of $SO_3$ generally lies between 1 and 6 percent by volume calculated on the volume of the carrier gas.

The beta-sultone preferably reacts with a nucleophile which may be methanol, ethanol, propanol, isopropanol, n-pentanol, triethylene glycol mono-methyl ether, ethanolamine, n-butylamine, sodium n-propyl thiolate, sodium ethoxide or sodium phenolate.

The beta-sultone can react with the nucleophile in several ways. Of these, two can generate surface active material, by

1. abstraction of a gamma-proton, resulting in the preparation of an alkene sulphonate,
2. substitution on the oxygen bearing beta-carbon atom, which is the desired reaction, resulting in the novel products of the invention. In many applications a combination of the latter with alkene sulphonate may

be used as surface active agents.

If desired the beta-substituted product is converted into a surface active ionic form by reaction with a base, e.g. sodium hydroxide. The invention further relates to beta-substituted sulphonic acids and/or sulphonates of the formula

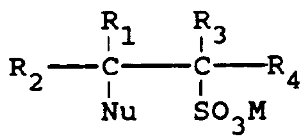 ,

wherein each of the groups $R_1$, $R_2$, $R_3$ and $R_4$ independently are linear or branched alkyl groups or hydrogen and the total number of carbon atoms of $R_1$, $R_2$, $R_3$ and $R_4$ is from 6 to 24, and at least one of $R_1$ and $R_2$ and one of $R_3$ and $R_4$ is an alkyl group, Nu is an aliphatic or aromatic (thio)ether radical, a (capped) polyethylene glycol- or polypropylene glycol ether radical, an amine radical or positively charged amine radical and M is hydrogen, an (earth) alkali metal, ammonium or a negative charge. Specifically may be mentioned that Nu may be

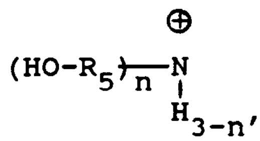

wherein n is an integer of 0 to 3 and $R_5$ is a straight chain or branched alkylene radical.

<u>EXAMPLE 1</u>

The sulphonation of $C_{18}$ internal olefin was performed in a glass falling film reactor, having a diameter of 0.5 cm and a length of 1 m. The $C_{18}$ internal olefin flowed along the inner part of the reactor wall as a flowing film in downward direction. Cocurrently with the olefin a $SO_3$ containing stream of dry nitrogen was fed into the reactor; the $SO_3$ containing nitrogen stream was prepared by evaporating liquid $SO_3$ at a carefully controlled rate in a heater. The following conditions were applied in the falling film sulphonation reactor:

| | |
|---|---|
| cooling water temperature: | 15 °C |
| $SO_3$/olefin ratio (mol/mol) | 1.2 |
| $SO_3$ in $N_2$ (% vol.) | 3 |
| olefin feed rate (mol/h) | 1.5 |

The cooling water, cooling the falling film sulphonation reactor, flowed countercurrently through the cooling jacket of the reactor. The sulphonation product leaving the falling film reactor was led through a cyclone to vent unreacted $SO_3$ and nitrogen. The subsequent reaction with a nucleophile was carried out batchwise by leading 40 g of beta-sultone containing product directly out of the cyclone into 80 g of monoethanol amine, that had been charged into a three-necked reaction flask equipped with a magnetic stirrer. The resulting mixture was subsequently allowed to stir overnight without external cooling. After that the total surfactant (active matter, AM) formed was determined on a sample withdrawn from the product mixture by direct two phase titration using hyamine under alkaline conditions using dichlorofluoresceine (Diederich indicator). The AM obtained in this way amounted 91% mol. Determination of the anionic surface active AM by direct two phase titration using hyamine under acid conditions with a mixed acid indicator yielded a much lower value (25% mol) demonstrating the betaine structure of the other 66% mol. Subsequently, the remainder of the product mixture was converted into the anionic form with an aqueous alcoholic NaOH solution. After that the unreacted olefins and apolar components were removed from the product mixture by three successive extractions with n-pentane. The resulting aqueous product mixture containing the surface active material was concentrated to constant weight using a rotary evaporator under reduced pressure, leaving a solid product mass. The molar ratio of alkene sulphonates and the desired betaines [beta-(monoethanolammonium) sulphonates] was determined by $^{13}C$ NMR spectroscopy: this ratio amounted to 25:75. It should be noted that the sulphonation of internal olefins in this way always yields an initial beta-sultone mixture containing approximately 10% mol of alkene sulphonic acids which do not participate in the subsequent reaction with nucleophiles. If the yield of the desired betaines is corrected accordingly (i.e. based on 100% beta-sultone intake), a yield of 77% mol is obtained.

EXAMPLE 2

Sulphonation of a $C_{13/14}$ internal olefin and subsequent reaction of the obtained beta-sultone with n-butyl amine was performed in the same way as described in Example 1. The following results were obtained:
AM (Diederich) : 92% mol
AM (mixed acid): 18% mol
betaine yield (by difference): 74% mol
Alkene sulphonate/betaine molar ratio by $^{13}C$ NMR: 20:80.
Corrected betaine yield: 82%.

EXAMPLES 3-9

Sulphonation of the internal olefin was performed in the same way as described in Example 1. The sultone containing product leaving the cyclone was led into 80 g of the alcohol (indicated in Table I) present in a three-necked reaction flask equipped with a magnetic stirrer. The resulting mixture was subsequently allowed to stir overnight without external cooling. Thereafter the total anionic surfactant formed was determined on an aliquot withdrawn from the product mixture, by direct two phase titration using hyamine under acid conditions with a mixed acid indicator. The total anionic active matter (AM) is indicated in Table I for each individual alcohol. Subsequently the remainder of the product mixture was neutralized to pH 7 with an aqueous alcoholic NaOH solution. After that the unreacted olefins and apolar components were removed from the product mixture by three successive extractions with n-pentane. The resulting aqueous alcoholic product mixture containing the anionic AM was concentrated to constant weight using a rotary evaporator under reduced pressure, leaving a solid product mass. The molar ratio of alkene sulphonates and the desired beta-substituted sulphonates was determined by $^{13}C$ NMR spectroscopy; the molar ratios obtained are given in Table I.

It should be noted that the sulphonation of internal olefins in this way always yields an initial beta-sultone mixture containing about 10% mol of alkene sulphonic acids which do not participate in subsequent reaction with nucleophiles. The last column in the table gives the yields of beta-substituted sulphonates calculated by taking into account that the mixture leaving the cyclone contained 10% mol of alkene sulphonates.

TABLE I

| Example | starting internal olefin | alcohol | total anionic surfactant (% mol) | alkene sulphonate/ beta-subst. sulpho- nate by $^{13}C$ NMR (mol/mol) | corrected yield (% mol) |
|---|---|---|---|---|---|
| 3 | $C_{13/14}$ | $CH_3OH$ | 76 | 15:85 | 72 |
| 4 | $C_{13/14}$ | $C_2H_5OH$ | 60 | 30:70 | 47 |
| 5 | $C_{18}$ | $C_2H_5OH$ | nd | 35:65 | nd |
| 6 | $C_{13/14}$ | $C_3H_7OH$ | 65 | 30:70 | 51 |
| 7 | $C_{13/14}$ | $i-C_3H_7OH$ | 46 | 35:65 | 28 |
| 8 | $C_{13/14}$ | $C_5H_{11}OH$ | 61 | 30:70 | 48 |
| 9 | $C_{13/14}$ | $CH_3(OCH_2CH_2)_3OH$ | 48 | nd | nd |

EXAMPLES 10-11

Sulphonation of the internal olefins (indicated in Table II) was performed in the same way as described in Example 1.

The sultone containing product leaving the cyclone was introduced into a three-necked reaction flask

equipped with a magnetic stirrer and containing 1,2 equivalents (calculated on acid intake ex cyclone) nucleophile in 80 g of solvent in a nitrogen atmosphere. The resulting mixture was subsequently stirred overnight without external cooling. After that the total anionic surfactant formed was determined on an aliquot withdrawn from the product mixture, by direct two phase titration using hyamine under acid conditions with a mixed acid indicator. The total anionic active matter (AM) is indicated in Table II for each individual nucleophile. Subsequently the remainder of the product mixture was extracted three times with n-pentane at pH of 7 in order to remove unreacted olefins and apolar components. The resulting aqueous alcoholic product mixture containing the anionic AM was concentrated to a solid product mass. The molar ratio of alkene sulphonates and beta-substituted sulphonates was determined by $^{13}C$ NMR spectroscopy.

EXAMPLE 12

Sulphonation of the $C_{18}$ internal olefin was performed in the same way as described in Example 1.

The sultone containing product leaving the cyclone was introduced into a three-necked reaction flask equipped with a magnetic stirrer and a reflux condenser, and containing 1.0 equivalents (calculated on acid intake ex cyclone) of lithium ethoxide (from n-butyllithium and ethanol in n-hexane) in a solvent mixture of 50 g n-hexane and 20 g anhydrous tetrahydrofuran under a nitrogen atmosphere. The resulting mixture subsequently was stirred for one hour at 35 °C and then was refluxed for 18 hours. After cooling the product mixture was analysed as indicated in Examples 10-11. The results are shown in Table II.

## TABLE II

| Example | internal olefin | nucleophile | solvent | total anionic surfactant (% mol) | alkene sulphonate/ beta subst. sulphonate by $^{13}C$ NMR (mol/mol) |
|---|---|---|---|---|---|
| 10 | $C_{13/14}$ | $C_3H_7SNa$ | $H_2O$ | 53 | nd |
| 11 | $C_{13/14}$ | ØONa | $H_2O$ | 76 | nd |
| 12 | $C_{18}$ | $C_2H_5OLi$ | n-hexane/ tetrahydro- furan | 90 | 20:80* |

* beta-ethoxy sulphonate:beta-hydroxy sulphonate 50:50

**Claims**

1. A process for the preparation of beta-substituted sulphonic acids and/or sulphonates which comprises reacting a beta-sultone of an internal olefin having the chemical formula

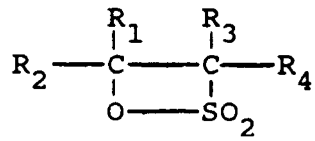 ,

wherein each of the groups $R_1$, $R_2$, $R_3$ and $R_4$ independently are linear or branched alkyl groups or hydrogen and the total number of carbon atoms of $R_1$, $R_2$, $R_3$ and $R_4$ is from 6 to 24, and at least one of $R_1$ and $R_2$ and one of $R_3$ and $R_4$ is an alkyl group, in the absence of dioxane with a nucleophile, with the proviso that the nucleophile is not $H_2O$ or hydroxide ion.

2. A process as claimed in claim 1, wherein the nucleophile is an aliphatic or aromatic (thio)alcohol or an alkoxide thereof, a (capped) polyethylene glycol or polypropylene glycol or the alkoxide thereof, an aliphatic or aromatic amine or an amide thereof, ammonia or a heterocyclic nitrogen compound.

3. A process as claimed in claim 1 or 2, wherein the nucleophile is methanol, ethanol, propanol, isopropanol, n-pentanol, triethylene glycol monomethyl ether, ethanolamine, n-butylamine, sodium n-propylthiolate, sodium ethoxide or sodium phenolate.

4. A process as claimed in any one of the claims 1-3, wherein the beta-sultone has been prepared by reacting in a film reactor an internal olefin having from 8 to 26 carbon atoms with a sulphonating agent, in a mol ratio of sulphonating agent to internal olefin of 1:1 to 1.25:1 while cooling the reactor with a cooling means having a temperature not exceeding 35 °C.

5. A process as claimed in claim 4, wherein ageing of the obtained beta-sultone is omitted or minimized.

6. Beta-substituted sulphonic acids and/or sulphonates of the formula

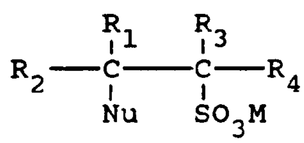 ,

wherein each of the groups $R_1$, $R_2$, $R_3$ and $R_4$ independently are linear or branched alkyl groups or hydrogen and the total number of carbon atoms of $R_1$, $R_2$, $R_3$ and $R_4$ is from 6 to 24 and at least one of $R_1$ and $R_2$ and one of $R_3$ and $R_4$ is an alkyl group, Nu is an aliphatic or aromatic (thio)ether radical, a (capped) polyethylene glycol or polypropylene glycol ether radical, an amine radical or positively charged amine radical and M is hydrogen, an (earth) alkali metal, ammonium or a negative charge.

7. Beta-substituted sulphonates as claimed in claim 6, wherein Nu is

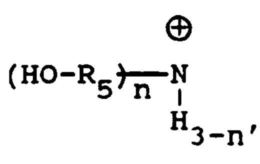

wherein n is an integer of 0 to 3 and $R_5$ is a straight chain or branched alkylene radical.

## Patentansprüche

1. Ein Verfahren zur Herstellung von beta-substituierten Sulphonsäuren und/oder Sulphonaten, umfassend das zur-Reaktionbringen eines beta-Sultons eines inneren Olefins mit der chemischen Formel

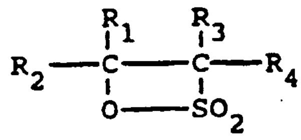

in der jede der Gruppen $R_1$, $R_2$, $R_3$ und $R_4$ unabhängig lineare oder verzweigte Alkylgruppen oder Wasserstoff sind und die Gesamtzahl an Kohlenstoffatomen von $R_1$, $R_2$, $R_3$ und $R_4$ im Bereich von 6 bis 24 liegt, und mindestens eine der Gruppen $R_1$ und $R_2$ und eine der Gruppen $R_3$ und $R_4$ eine Alkylgruppe ist, mit einem Nukleophil in Abwesenheit von Dioxan, mit der Maßgabe, daß das Nukleophil nicht $H_2O$ oder ein Hydroxidion ist.

2. Ein Verfahren, wie in Anspruch 1 beansprucht, in dem das Nukleophil ein aliphatischer oder aromatischer (Thio)alkohol oder ein Alkoxid davon, ein mit Endgruppen versehenes (capped) Polyethylenglycol oder Polypropylenglycol oder dessen Alkoxid, ein aliphatisches oder aromatisches Amin oder ein Amid davon, Ammoniak oder eine heterocyclische Stickstoffverbindung ist.

3. Ein Verfahren, wie in Anspruch 1 oder 2 beansprucht, in dem das Nukleophil Methanol, Ethanol, Propanol, Isopropanol, n-Pentanol, Triethylenglycolmonomethylether, Ethanolamin, n-Butylamin, Natrium-n-propylthiolat, Natriumethoxid oder Natriumphenolat ist.

4. Ein Verfahren, wie in irgendeinem der Ansprüche 1 bis 3 beansprucht, in dem das beta-Sulton hergestellt wurde durch das zur-Reaktion-bringen eines inneren Olefins mit 8 bis 26 Kohlenstoffatomen mit einem sulphonierenden Mittel in einem Filmreaktor, wobei das Molverhältnis des sulphonierenden Mittels zum inneren Olefin von 1:1 bis 1,25:1 betrug, während der Reaktor mit einem Kühlmittel mit einer Temperatur nicht über 35°C gekühlt wurde.

5. Ein Verfahren, wie in Anspruch 4 beansprucht, in dem das Altern des erhaltenen beta-Sultons unterlassen oder minimiert wird.

6. Beta-substituierte Sulphonsäuren und/oder Sulphonate der Formel

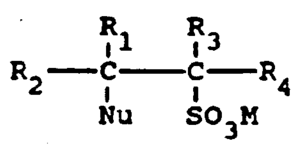

in der jede der Gruppen $R_1$, $R_2$, $R_3$ und $R_4$ unabhängig lineare oder verzweigte Alkylgruppen oder Wasserstoff sind und die Gesamtzahl an Kohlenstoffatomen von $R_1$, $R_2$, $R_3$ und $R_4$ im Bereich von 6 bis 24 liegt und mindestens eine der Gruppen $R_1$ und $R_2$ und eine der Gruppen $R_3$ und $R_4$ eine Alkylgruppe ist, Nu ein aliphatisches oder aromatisches (Thio)etherradikal, ein mit Endgruppen versehenes (capped) Polyethylenglycol- oder Polypropylenglycoletherradikal, ein Aminradikal oder ein positiv geladenes Aminradikal und M Wasserstoff, ein (Erd-)alkalimetall, Ammonium oder eine negative Ladung ist.

7. Beta-substituierte Sulphonate, wie in Anspruch 6 beansprucht, in denen Nu

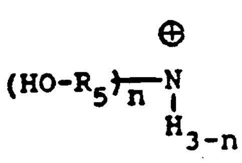

ist, wobei n eine ganze Zahl von 0 bis 3 und $R_5$ ein geradkettiges oder verzweigtes Alkylenradikal ist.

**Revendications**

1. Un procédé pour la préparation d'acides sulfoniques et/ou de sulfonates bêta-substitués, qui comprend la réaction d'une bêta-sultone d'une oléfine interne ayant la formule chimique

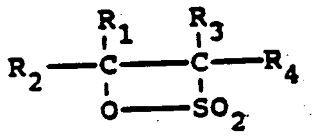

dans laquelle chacun des groupes $R_1$, $R_2$, $R_3$ et $R_4$ indépendamment est un groupe alkyle linéaire ou ramifié ou de l'hydrogène et le nombre total d'atomes de carbone de $R_1$, $R_2$, $R_3$ et $R_4$ est compris entre 6 et 24, et au moins un de $R_1$ et $R_2$ ainsi qu'un de $R_3$ et $R_4$ est un groupe alkyle, en l'absence de dioxane, avec un nucléophile, avec la condition que le nucléophile n'est pas $H_2O$ ou un ion hydroxyde.

2. Un procédé selon la revendication 1, dans lequel le nucléophile est un (thio)alcool aliphatique ou aromatique ou un alcoolate qui en dérive, un polyéthylène glycol ou polypropylène glycol (coiffé) ou un alcoolate qui en dérive, une amine aliphatique ou aromatique ou un amide qui en dérive, l'ammoniac ou un composé azoté hétérocyclique.

3. Un procédé selon la revendication 1 ou 2, dans lequel le nucléophile est le méthanol, l'éthanol, le propanol, l'isopropanol, le n-pentanol, l'éther monométhylique du triéthylène glycol, le n-propyl-thiolate de sodium, l'éthylate de sodium ou le phénolate de sodium.

4. Un procédé selon l'une quelconque des revendications 1-3, dans lequel on a préparé la bêta-sultone en faisant réagir dans un réacteur à film une oléfine interne ayant de 8 à 26 atomes de carbone avec un agent de sulfonation, dans un rapport molaire de l'agent de sulfonation à l'oléfine interne compris entre 1:1 et 1,25:1, tout en refroidissant le réacteur avec un agent de refroidissement ayant une température qui ne dépasse pas 35°C.

5. Un procédé selon la revendication 4, dans lequel le vieillissement de la bêta-sultone obtenue est omis ou réduit au minimum.

6. Acides sulfoniques et/ou sulfonates bêta-substitués de la formule

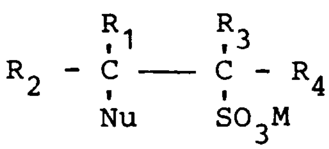

dans laquelle chacun des groupes $R_1$, $R_2$, $R_3$ et $R_4$ indépendamment est un groupe alkyle linéaire ou ramifié ou de l'hydrogène et le nombre total d'atomes de carbone de $R_1$, $R_2$, $R_3$ et $R_4$ est compris entre 6 et 24 et au moins un de $R_1$ et $R_2$ ainsi qu'un de $R_3$ et $R_4$ est un groupe alkyle, Nu est un radical de (thio)éther aliphatique ou aromatique, un radical d'éther de polyéthylène glycol ou de polypropylène glycol (coiffé), un radical d'amine ou un radical d'amine chargé positivement et M est l'hydrogène, un métal alcalin ou alcalino-terreux, l'ammonium ou une charge négative.

7. Sulfonates bêta-substitués selon la revendication 6, dans lesquels Nu est

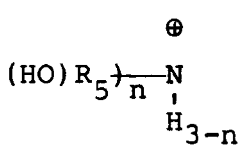

où n est un nombre entier de 0 à 3 et $R_5$ est un groupe alkylène à chaîne droite ou ramifié.